(19) **Europäisches Patentamt / European Patent Office / Office européen des brevets**

(11) **EP 1 827 536 B1**

(12) **EUROPEAN PATENT SPECIFICATION**

(45) Date of publication and mention
of the grant of the patent:
**30.06.2010 Bulletin 2010/26**

(51) Int Cl.:
***A61M 5/168*** *(2006.01)*

(21) Application number: **04806608.8**

(22) Date of filing: **21.12.2004**

(86) International application number:
**PCT/IB2004/052880**

(87) International publication number:
**WO 2006/067555 (29.06.2006 Gazette 2006/26)**

(54) **LIQUID DOSING ARRANGEMENT**

ANORDNUNG ZUM DOSIEREN VON FLÜSSIGKEITEN

AGENCEMENT DE DOSAGE LIQUIDE

(84) Designated Contracting States:
**DE FR**

(43) Date of publication of application:
**05.09.2007 Bulletin 2007/36**

(73) Proprietor: **Maquet Critical Care AB**
**171 95 Solna (SE)**

(72) Inventor: **SLETTENMARK, Bruno**
**S-175 69 Järfälla (SE)**

(74) Representative: **Herbjörnsen, Rut et al**
**Albihns.Zacco**
**Valhallavägen 117**
**Box 5581**
**114 85 Stockholm (SE)**

(56) References cited:
**US-A- 4 340 050     US-A- 4 626 243**
**US-A- 4 976 687     US-A- 6 129 702**
**US-A1- 2002 156 464**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the invention**

The present invention relates to a liquid dosing arrangement, and more specifically to an arrangement for maintaining flow accuracy without the use of a flow sensor when an injection valve is used for dosing liquids.

**Background and prior art**

There is often a need for dosing liquids with good accuracy, especially in the field of medicine, e.g. during intravenous infusion or epidural anesthesia. A number of different systems exist on the market, e.g. syringe infusion pumps, peristaltic pumps and hydrostatic infusion devices. All these methods have both advantages and drawbacks with respect to for example performance, security, complexity and pricing. Syringe infusion pumps for example give good accuracy, but are quite expensive. It is therefore advantageous to instead use a simple and cheap injection valve for dosing liquid.

An injection valve releases liquid in short pulses from a pressurized liquid source. The flow of liquid can be controlled by varying the pulse frequency and/or the pulse width of the liquid pulses. The flow however varies significantly, depending on parameters such as the ambient temperature, the inner diameter of the tubing, the radius of curvature of the tubing, counter pressure from the patient, or occlusions in the catheters. The ambient temperature causes the viscosity of the liquid to change - the viscosity of water for example decreases 50% when the temperature changes from 10°C to 40°C. The counter pressure from the patient depends on parameters such as blood pressure, and also varies due to changes in hydrostatic pressure as the patient moves. Therefore an accurate flow sensor is required in order for the flow to be controlled. Accurate flow sensors are however quite expensive and have a limited flow range, and since they are also difficult to sterilize, they are hardly suitable for the intended applications.

US 4 976 687 discloses a liquid dosing arrangement for dosing intravenous fluids according to the preamble of claim 1, comprising a liquid supply system having a first and a second pressure sensor and an injection valve located therebetween. The liquid is supplied through a restriction in the injection valve. The arrangement of D1 comprises a flow restrictor which provides a constant time delay for the fluid passing therethrough.

There is therefore a need for a method and an apparatus for using an injection valve for accurately dosing liquids without the use of an accurate flow sensor.

**Summary of the invention**

To achieve this objective the present invention provides a liquid dosing arrangement for dosing a liquid to be delivered to a patient as defined in claim 1. The liquid dosing arrangement comprises a liquid supply system in which a first pressure sensor in use is measuring a first pressure $p_1$; a liquid delivery system in which a second pressure sensor in use is measuring a second pressure $p_2$; and an injection valve, located between the liquid supply system and the liquid delivery system, the liquid being supplied through a restricting member of the injection valve, and being let through the restricting member by a pulsed opening and closing valve mechanism with opening pulses having frequency $f_o$ and width $t_o$, and the pressure drop over the injection valve being $\Delta p = p_1 - p_2$; **characterized in that** the pressure drop $\Delta p$, and the dimensions of the restricting member in the injection valve, are selected to make the velocity $v$ of the liquid flowing through the restricting member high enough to make the flow $\Phi$ of liquid through the liquid delivery system essentially independent of the viscosity of the liquid.

The invention thereby makes it possible, with a comparatively simple and cheap device, to achieve an accurate flow control irrespective of downstream counter pressure as well as variations in temperature and geometric variations of the tubing, and without the use of accurate flow sensors.

In a preferred embodiment, the restricting member in the injection valve is an orifice in an orifice plate. The diameter of this orifice is preferably less than 300 $\mu$m. If a higher flow rate is desirable several orifices may be used, each with a diameter of typically less than 300 $\mu$m.

The liquid dosing arrangement preferably further comprises an expansion chamber downstream of the injection valve, preferably located immediately downstream of the injection valve.

A low pass filter can be arranged to filter the signal from the second pressure sensor. In a preferred embodiment this signal is then fed to a pressure regulating system which is arranged to feed a regulating signal to a pressure regulator in order to regulate the first pressure $p_1$ in the liquid supply system.

The liquid dosing arrangement preferably further comprises an alarm which is arranged to be activated when the second pressure $p_2$ measured by the second pressure sensor exceeds a certain preset value $p_{max}$, or when the flow measured by a simple monitoring flow sensor in the liquid supply system deviates beyond certain preset values.

The present invention further provides an apparatus for intravenous infusion comprising a liquid dosing arrangement according to the invention.

A method of dosing liquid is also presented. It comprises the steps of providing a liquid dosing arrangement comprising a liquid supply system, a liquid delivery system and an injection valve; measuring a first pressure $p_1$ in the liquid supply system by the use of a first pressure sensor; and measuring a second pressure $p_2$ in the liquid delivery system by the use of a second pressure sensor; characterized in selecting the pressure drop $\Delta p = p_1 - p_2$ over the injection valve, and the dimensions of the restricting member in the injection valve, so that the velocity $v$ of the liquid flowing through the restricting member becomes high enough to make the flow $\Phi$ of liquid through the liquid delivery system essentially independent of the viscosity of the liquid.

The method of dosing liquid preferably further comprises the step of providing an expansion chamber downstream of the injection valve, preferably immediately downstream of the injection valve.

In a preferred embodiment, the method further comprises the steps of filtering the signal from the second pressure sensor in a low pass filter; feeding the signals from the first and second pressure sensors to a pressure regulating system; and feeding the output of the regulating system as a regulating signal to a pressure regulator in order to regulate the first pressure $p_1$ in the liquid supply system.

The method preferably further comprises the step of activating an alarm when the second pressure $p_2$ measured by the second pressure sensor exceeds a certain preset value $p_{max}$, or when the flow measured by a simple monitoring flow sensor in the liquid supply system deviates beyond certain preset values.

### Brief description of the drawings

Reference is now made to the attached drawings for a better understanding of the present invention and its examples and embodiments, wherein:

Figure 1 schematically illustrates a liquid dosing system comprising an injection valve according to one embodiment of the present invention.
Figure 2a schematically illustrates an orifice plate which is a part of the injection valve in figure 1.
Figure 2b is a side view of the orifice plate in figure 2a.

### Detailed description of preferred embodiments

The pressure $\Delta p$ over an injection valve depends on the density and the viscosity of the liquid flowing through the valve in the following way:

$$\Delta p = k_1 * \frac{\rho * v^2}{2} + k_2 * \eta(T) * v$$

$v = $ the velocity of the liquid flowing through the restricting member

$\rho = $ the density of the liquid (which depends very little on the temperature)

$\eta(T) = $ the dynamic viscosity of the liquid (which depends very strongly on the temperature)

$k_1, k_2 = $ constants ($k_1$ is normally about 1-1,5, assuming that the kinetic energy is lost in eddies downstream the abrupt restriction)

For values of $v$ which are high enough, the viscosity part becomes negligible, since it only depends on $v$, while the kinetic pressure drop depends on $v^2$. For values of $v$ which are high enough, the pressure drop $\Delta p$ thus becomes virtually independent of the viscosity $\eta(T)$, and accordingly also of the temperature T.

For high enough values of $v$ we therefore have:

$$\Delta p = k_1 * \frac{\rho * v^2}{2} \quad \Rightarrow \quad v = \sqrt{\frac{2 * \Delta p}{k_1 * \rho}}$$

The flow $\Phi$ through an injection valve depends on the velocity $v$ of the liquid and the diameter $d_1$ of the restricting member in the following way:

$$\Phi = \frac{\pi * d_1^2}{4} * v = \frac{\pi * d_1^2 * \sqrt{2}}{4 * \sqrt{k_1}} * \sqrt{\frac{\Delta p}{\rho}}$$

Since the density $\rho$ of the liquid is almost constant regardless of the temperature, $\Phi$ thus becomes proportional to $\sqrt{\Delta p}$ , which can be kept constant by a very simple form of pressure regulation. For high enough values of $v$, $\Phi$ is therefore independent of all temperature and time dependent pressure drops downstream of the injection valve. Also, the fact that $\Phi$ is proportional to $\sqrt{\Delta p}$ results in that a regulating error in $\Delta p$ of x% only leads to an error in $\Phi$ of x/2%.

A high velocity $v$ of the liquid through the restricting member in the injection valve is accomplished by letting the restricting member have a small diameter, and letting $\Delta p$ be high. $\Delta p$ should therefore be regulated to a value high enough, and the diameter of the restricting member selected to be small, with the additional condition that a desired maximum flow $\Phi_{max}$ can be accomplished and that certain practical additional conditions are met, such as the limitation that $\Delta p$ can never be higher than the available pressure $p_{in}$ of the employed pressure source.

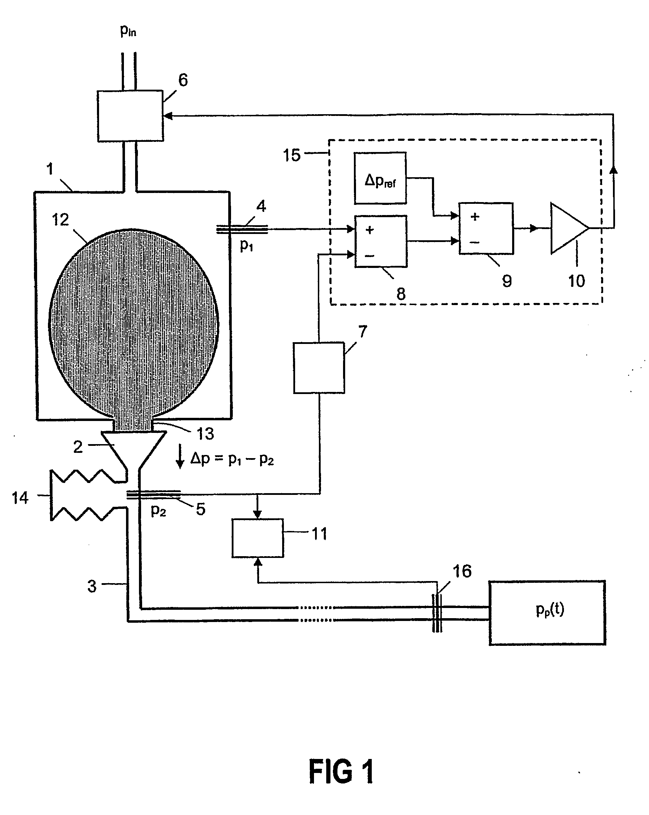

The practical application of this will now be described with reference to figure 1. Figure 1 shows a liquid dosing system comprising a pressurized liquid source 1, an injection valve 2 and a liquid delivery system 3. The pressurized liquid source 1 can for example be an elastic container 12 under pressure in the form of a so called bag-in-bottle. The source of the driving pressure $p_{in}$ can for example be the compressed air that is usually available in tapping sources in the walls in hospitals, but of course any suitable pressure source can be used. The driving pressure $p_{in}$ is in hospital environments usually about 2 - 8 Bar. It is possible to use other methods of liquid feeding, such as mechanical pumps or other types of mechanical pressurizing means, instead of a bag-in-bottle. However, the described bag-in-bottle type liquid source is preferred due to its simplicity and to the ease of maintaining the sterility of the liquid in critical applications.

The injection valve 2 releases liquid in pulses having frequency $f_o$ and width $t_o$ from the pressurized liquid source 1. The pressure drop over the injection valve 2 is $\Delta p = p_1 - p_2$. If the driving pressure $p_{in}$ is constant and no regulation is effected, the pressure $p_2$ varies significantly, depending on parameters such as the ambient temperature, the radius of curvature of the tubing, counter pressure from the patient, or occlusions in the catheters. This results in an undesirable strong variation of the flow $\Phi$, since this as explained above depends on the pressure drop $\Delta p$.

According to the invention the liquid dosing system therefore further comprises pressure sensors 4 and 5. Pressure sensor 4 measures the pressure $p_1$ in the liquid dosing system 1, and pressure sensor 5 measures the pressure $p_2$ immediately after the injection valve 2. In the configuration shown in figure 1 the bag 12 must be very pliable and its wall material not stretched in order for the pressure sensor 4 to measure the correct liquid pressure inside the liquid container. The signal from pressure sensor 5 is filtered through a low pass filter 7, and then fed to a pressure regulating system 15 together with the signal from pressure sensor 4. The pressure regulating system 15 outputs a regulation signal to a pressure regulator 6 which regulates the pressure $p_1$ in order to keep $\Delta p = p_1 - p_2$ constant and equal to a certain preset value $\Delta p_{ref}$. $\Delta p_{ref}$ is preferably selected to the highest possible value which is practical in the chosen application. In a hospital environment a suitable $\Delta p_{ref}$ can for example be 3 Bar.

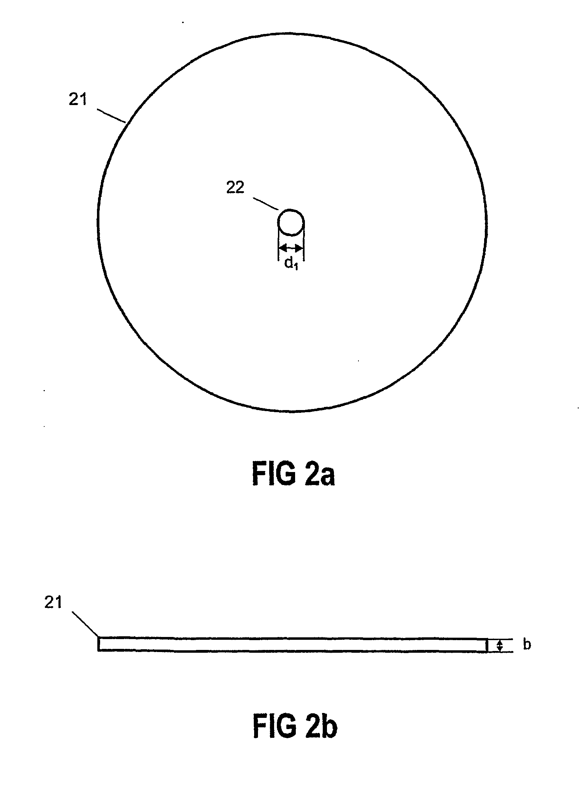

The injection valve 2 preferably has a restricting member in the form of at least one circular hole 22 drilled in an orifice plate 21, as shown in figure 2a, but other restricting members can also be used. The restricting member should be "short", which is accomplished by the orifice plate 21 being thin, i.e. the thickness b in figure 2b being small. A thin restricting orifice plate 21 also reduces any contribution to the pressure $\Delta p$ from the viscosity part. The diameter $d_1$ of the hole(s) 22 in the orifice plate 21 should as explained above be as small as possible, with respect to parameters such as $\Delta p$, the desired maximum flow $\Phi_{max}$, the characteristics of the liquid, and other practical considerations. The hole(s) 22 can of course have any suitable shape, not necessarily circular. The flow $\Phi$ is accomplished by opening the injection valve 2 fully during short pulses, having frequency $f_o$ and width $t_o$. Variation of the flow is accomplished by varying either $t_o$ or $f_o$, or alternatively a combination of both.

The liquids used in hospitals are often water-based diluted solutions. Within the limitations explained above the velocity $v$ of the liquid will be the same for the same pressure drop $\Delta p_{ref}$, frequency $f_o$ and width $t_o$. If for a specific type of injection valve the thickness b of the orifice plate is about 0,2 mm, $\Delta p_{ref}$ is set to 3 Bar and the valve is opened for 2 ms every 10 seconds, the velocity $v$ will be 20 m/s. If the injection valve has one hole having a diameter $d_1$ of 100 $\mu$m, $\Phi_{max}$ will then be about 160 $\mu$l/s and $\Phi_{min}$ will be about 0,03 $\mu$l/s.

If the diameter $d_1$ of the hole is only 50 $\mu$m, $\Phi_{max}$ will only be about 40 $\mu$l/s and $\Phi_{min}$ will be about 0,01 $\mu$l/s.

If the diameter $d_1$ of the hole is 200 $\mu$m, $\Phi_{max}$ will be about 630 $\mu$l/s and $\Phi_{min}$ will be about 0,13 $\mu$l/s.

If the diameter $d_1$ of the hole is 300 $\mu$m, $\Phi_{max}$ will be about 1400 $\mu$l/s and $\Phi_{min}$ will be about 0,28 $\mu$l/s.

If the injection valve instead has four holes, each having a diameter $d_1$ of 100 $\mu$m, $\Phi_{max}$ will be about 630 $\mu$l/s and $\Phi_{min}$ will be about 0,13 $\mu$l/s.

These values are of course just examples of working embodiments, and they are in no way limiting to the scope of the invention. There are many different types of injection valves and they all have specific characteristics, for example concerning the amount of viscous pressure drop in the valve mechanism, and the type of liquid used also affects the flow, so in practice an empiric optimization of the relevant parameters must be done, based on the above explained principles.

It is also possible to use an injection valve having an orifice plate with a hole having a diameter $d_1$ which is larger than 300 $\mu$m, but with a larger diameter the advantages of the claimed solution become less apparent.

A problem with liquid delivery systems such as the one shown in figure 1 is that they normally comprise long plastic tubing with a small inner diameter and rigid walls. This causes them to have a very high analog "inductance", i.e. "resistance" to quick flow changes.

The pressure drop $p_2$ over the tubing is a function of the flow change according to:

$$\mathrm{p}_2 = \dot{\Phi} * L \qquad \text{where} \qquad L = \frac{4 * \rho * l}{\pi * \mathrm{d}_2^2}$$

$d_2 =$     the inner diameter of the tubing
$l =$     the length of the tubing
$\rho =$     the density of the liquid

If the pulse time $t_o$ is short, this results in that virtually no liquid flow at all has time to occur, and $\Phi$ thus does not become proportional to $t_o$ when the pulse time is increased.

One way of counteracting this effect is to place an expansion chamber 14 immediately downstream of the valve 2, with a compliance of $C = \Delta V / \Delta p$, adapted so that the pressure increase $\Delta p$ becomes sufficiently small for the volume $\Delta V$ that is obtained at the longest time $t_o$ that will be used in the pulse train. An expansion chamber 14 with a suitable shape and adequate compliance (caused by e.g. a spring load or the elasticity of the chamber itself) is therefore shown in figure 1 positioned immediately downstream of the injection valve 2, in order to absorb the pressure transients from the pulsed operation. Now, the effect of the inertia of the liquid in the tubing is eliminated and the mean flow through the tubing will be essentially proportional to the pulse width. Any remaining ripple in the pressure signal $p_2$ is filtered away in the low pass filter 7, which should have a long time constant (typically 1 - 10 s).

Viscous pressure drop or kinetic pressure drop between the container 12 containing the liquid and the injection valve 2 is also undesirable. Therefore the tubing 13 from the container 12 to the injection valve 2 should be as short as possible and have a sufficiently large inner diameter. Otherwise the pressure regulator 6 cannot manage to keep $\Delta p$ at a constant value.

Pressure sensors 4 and 5 are preferably of a type that is simple, cheap and easy to clean. They may also be of a disposable type.

The pressure regulating system 15 can be designed in any suitable way, but preferably has a comparator device 8 which calculates the pressure drop $\Delta p$, and another comparator device 9 which compares the pressure drop $\Delta p$ with the preset value $\Delta p_{ref}$. It is advantageous to also include an amplifying device 10 for amplifying the error signal before feeding it to the pressure regulator 6.

In a preferred embodiment, the liquid dosing arrangement further comprises an occlusion alarm 11, which is activated when $p_2$ exceeds a certain preset value $p_{max}$. The occlusion alarm 11 can be used to interrupt the flow by lowering $p_1$ to zero, discontinue the pulsing of the valves, etc.

When a liquid dosing arrangement according to the invention is used for infusion, the pressure sensor 5 and the occlusion alarm 11 are already present in the system, since this is a requirement for infusion. The pressure sensor 5 is normally placed immediately after the valve.

Some kind of flow alarm 11 at instances such as major valve leakage or errors in the regulation, which causes the flow measured by a flow sensor 16 in the liquid supply system 3 to deviate beyond certain preset values, can also be required for certain applications. In this case, a very simple type of flow sensor 16 of pressure drop type (with lesser accuracy) can be used for this function. The alarm 11 can be the same alarm as the one used for the occlusion alarm, or be a separate device.

The present invention has been described with non-limiting examples and embodiments. It is to be understood that only the attached claims define all possible embodiments for a person skilled in the art.

**Claims**

1. A liquid dosing arrangement for dosing a liquid to be delivered to a patient, comprising:

   a liquid supply system (1), in which a first pressure sensor (4) in use is measuring a first pressure $p_1$;
   a liquid delivery system (3), in which a second pressure sensor (5) in use is measuring a second pressure $p_2$; and
   an injection valve (2), located between the liquid supply system (1) and the liquid delivery system (3), which is arranged so that the liquid is supplied through a restricting member (22) in the injection valve (2) and let through the restricting member (22) by a pulsed opening and closing valve mechanism with opening pulses having frequency $f_o$ and width $t_o$, wherein the pressure drop $\Delta p$ over the injection valve (2) is $\Delta p = p_1 - p_2$; and a pressure regulator (6) for regulating the first pressure $p_1$;
   **characterized in that**
   the pressure $p_1$ is regulated in use to keep the pressure drop $\Delta p$ constant and equal to a certain preset value $\Delta p_{ref}$, and the dimensions (b, $d_1$) of the restricting member (22) in the injection valve (2), are selected to make the velocity $v$ of the liquid flowing through the restricting member (22) high enough to make the flow $\Phi$ of liquid through the liquid delivery system essentially independent of the viscosity of the liquid.

2. The liquid dosing arrangement according to claim 1, wherein the restricting member (22) in the injection valve (2) is at least one orifice (22) in an orifice plate (21).

3. The liquid dosing arrangement according to claim 2, wherein the restricting member (22) in the injection valve (2) is a plurality of orifices (22) in an orifice plate (21).

4. The liquid dosing arrangement according to any one of claims 2 - 3, wherein the at least one orifice (22) is approximately circular, and has a diameter $d_1$ of less than 300 $\mu$m.

5. The liquid dosing arrangement according to any one of claims 1 - 4, further comprising an expansion chamber (14) downstream of the injection valve (2).

6. The liquid dosing arrangement according to any one of claims 1 - 5, further comprising a pressure regulating system (15), feeding the signals from the first and second pressure sensors (4,5) to said pressure regulating system (15), which is arranged to feed a regulating signal to a pressure regulator (6) , which regulates the first pressure $p_1$ in the liquid supply system in order to keep $\Delta p$ constant.

7. The liquid dosing arrangement according to any one of claims 1 - 6, further comprising a low pass filter (7), which is arranged to filter the signal from the second pressure sensor (5).

8. The liquid dosing arrangement according to any one of claims 1 - 7, wherein the pressure regulating system (15) comprises a first comparator device (8), to which the signals from the pressure sensors (4,5) are fed, and which calculates the pressure drop $\Delta p$, and a second comparator device (9), which compares the pressure drop $\Delta p$, generated by the first comparator device (8), with a preset reference value $\Delta p_{ref}$ for the pressure drop, wherein the output from said second comparator device (9) represents the regulating signal for the pressure regulator (6).

9. The liquid dosing arrangement according to any one of claims 1 - 8, further comprising an alarm (11) which is arranged to be activated when the second pressure $p_2$ measured by the second pressure sensor (5) exceeds a certain preset value $p_{max}$.

10. The liquid dosing arrangement according to any one claims 1 - 9, further comprising an alarm (11) which is arranged to be activated when the flow measured by a flow sensor (16) in the liquid supply system (3) deviates beyond certain preset values.

11. An apparatus for intravenous infusion comprising a liquid dosing arrangement according to any one of claims 1 - 10.

**Patentansprüche**

1. Flüssigkeitsdosieranordnung zum Dosieren einer Flüssigkeit, die einem Patienten zuzuführen ist, umfassend:

ein Flüssigkeitsbereitstellungssystem (1), in dem ein erster Drucksensor (4) in Benutzung einen ersten Druck $p_1$ misst;

ein Flüssigkeitszuführungssystem (3), in dem ein zweiter Drucksensor (5) in Benutzung einen zweiten Druck $p_2$ misst; und

ein Einspritzventil (2), das zwischen dem Flüssigkeitsbereitstellungssystem (1) und dem Flüssigkeitszuführungssystem (3) angeordnet ist, das so ausgestaltet ist, dass die Flüssigkeit durch ein Begrenzungselement (22) in dem Einspritzventil (2) bereitgestellt wird und durch einen gepulsten Öffnungs- und Schließventilmechanismus durch das Begrenzungselement (22) gelassen wird, wobei Öffnungspulse Frequenz $f_0$ und Weite $t_0$ haben, wobei der Druckabfall $\Delta_p$ über das Einspritzventil (2) $\Delta_p = p_1-p_2$ ist; und einen Druckregulierer (6) zum Regulieren des ersten Drucks $p_1$;

**dadurch gekennzeichnet, dass** der Druck $p_1$ in Benutzung reguliert wird, um den Druckabfall $\Delta p$ konstant und gleich einem bestimmten voreingestellten Wert $\Delta p_{ref}$ zu halten; und die Maße (b, $d_1$) des Begrenzungselements (22) in dem Einspritzventil (2) ausgewählt sind, um die Geschwindigkeit v der Flüssigkeit, die durch das Begrenzungselement (22) strömt, hoch genug zu erzeugen, um die Strömung $\Phi$ von Flüssigkeit durch das Flüssigkeitszuführungssystem im Wesentlichen unabhängig von der Viskosität der Flüssigkeit zu machen.

2.  Flüssigkeitsdosieranordnung nach Anspruch 1, wobei das Begrenzungselement (22) in dem Einspritzventil (2) zumindest eine Mündung (22) in einer Mündungsplatte (21) ist.

3.  Flüssigkeitsdosieranordnung nach Anspruch 2, wobei das Begrenzungselement (22) in dem Einspritzventil (2) eine Mehrzahl von Mündungen (22) in einer Mündungsplatte (21) ist.

4.  Flüssigkeitsdosieranordnung nach einem der Ansprüche 2 bis 3, wobei die zumindest eine Mündung (22) etwa kreisförmig ist und einen Durchmesser $d_1$ von weniger als 300 $\mu$m hat.

5.  Flüssigkeitsdosieranordnung nach einem der Ansprüche 1 bis 4, ferner umfassend eine Expansionskammer (14) stromabwärts des Einspritzventils (2).

6.  Flüssigkeitsdosieranordnung nach einem der Ansprüche 1 bis 5, ferner umfassend ein Druckreguliersystem (15), das die Signale von dem ersten und zweiten Drucksensor (4, 5) in das Druckreguliersystem (15) speist, das ausgestaltet ist, um ein Regulationssignal in einen Druckregulierer (6) zu speisen, der den ersten Druck $p_1$ in dem Flüssigkeitsbereitstellungssystem reguliert, um $\Delta p$ konstant zu halten.

7.  Flüssigkeitsdosieranordnung nach einem der Ansprüche 1 bis 6, ferner umfassend ein Tiefpassfilter (7), das ausgestaltet ist, um das Signal von dem zweiten Drucksensor (5) zu filtern.

8.  Flüssigkeitsdosieranordnung nach einem der Ansprüche 1 bis 7, wobei das Druckregulierungssystem (15) eine erste Vergleichseinrichtung (8), in die die Signale von den Drucksensoren (4, 5) eingespeist werden und die den Druckabfall $\Delta p$ berechnet, und eine zweite Vergleichseinrichtung (9) umfasst, die den Druckfall $\Delta p$, der durch die erste Vergleichseinrichtung (8) erzeugt wurde, mit einem voreingestellten Bezugswert $\Delta p_{ref}$ für den Druckabfall vergleicht, wobei die Ausgabe von der zweiten Vergleichseinrichtung (9) das Regulationssignal für den Druckregulierer (6) repräsentiert.

9.  Flüssigkeitsdosieranordnung nach einem der Ansprüche 1 bis 8, ferner umfassend einen Alarm (11), der ausgestaltet ist, um aktiviert zu werden, wenn der zweite Druck $p_2$, der durch den zweiten Drucksensor (5) gemessen wurde, einen bestimmten voreingestellten Wert $p_{max}$ überschreitet.

10. Flüssigkeitsdosieranordnung nach einem der Ansprüche 1 bis 9, ferner umfassend einen Alarm (11), der ausgestaltet ist, um aktiviert zu werden, wenn die Strömung, die durch einen Strömungssensor (16) in dem Flüssigkeitsbereitstellungssystem (3) gemessen wurde, über bestimmte voreingestellte Werte hinaus abweicht.

11. Vorrichtung zur intravenösen Infusion umfassend eine Flüssigkeitsdosieranordnung nach einem der Ansprüche 1 bis 10.

**Revendications**

1. Agencement de dosage de liquide pour doser un liquide à délivrer à un patient, comprenant :

   un système d'alimentation en liquide (1), dans lequel un premier capteur de pression (4), en utilisation, mesure une première pression $p_1$ ;
   un système de distribution de liquide (3), dans lequel un deuxième capteur de pression (5), en utilisation, mesure une deuxième pression $p_2$ ; et
   une vanne d'injection (2), située entre le système d'alimentation en liquide (1) et le système de distribution de liquide (3), qui est agencée de sorte que le liquide soit fourni à travers un élément de limitation (22) dans la vanne d'injection (2) et sorti à travers l'élément de limitation (22) par un mécanisme de vanne à ouverture et fermeture pulsées dont les impulsions d'ouverture ont une fréquence $f_0$ et une durée $t_0$, dans lequel la chute de pression $\Delta p$ sur la vanne d'injection (2) est $\Delta p = p_1 - p_2$ ; et
   un régulateur de pression (6) pour réguler la première pression $p_1$ ;
   **caractérisé en ce que**
   la pression $p_1$ est régulée en utilisation pour maintenir la chute de pression $\Delta p$ constante et égale à une certaine valeur prédéterminée $\Delta p_{ref}$, et les dimensions (b, $d_1$) de l'élément de limitation (22) dans la vanne d'injection (2) sont sélectionnées pour que la vitesse u du liquide s'écoulant à travers l'élément de limitation (22) soit suffisamment élevée pour que l'écoulement $\Phi$ de liquide à travers le système de distribution de liquide soit essentiellement indépendant de la viscosité du liquide.

2. Agencement de dosage de liquide selon la revendication 1, dans lequel l'élément de limitation (22) dans la vanne d'injection (2) consiste en au moins un orifice (22) dans une plaque à orifices (21).

3. Agencement de dosage de liquide selon la revendication 2, dans lequel l'élément de limitation (22) dans la vanne d'injection (2) consiste en une pluralité d'orifices (22) dans une plaque à orifices (21).

4. Agencement de dosage de liquide selon l'une quelconque des revendications 2 à 3, dans lequel ledit au moins un orifice (22) est à peu près circulaire, et a un diamètre $d_1$ inférieur à 300 $\mu$m.

5. Agencement de dosage de liquide selon l'une quelconque des revendications 1 à 4, comprenant en outre une chambre d'expansion (14) en aval de la vanne d'injection (2).

6. Agencement de dosage de liquide selon l'une quelconque des revendications 1 à 5, comprenant en outre un système de régulation de pression (15), délivrant les signaux provenant des premier et deuxième capteurs de pression (4, 5) audit système de régulation de pression (15), qui est agencé pour délivrer un signal de régulation à un régulateur de pression (6), qui régule la première pression $p_1$ dans le système d'alimentation en liquide afin de maintenir $\Delta p$ constante.

7. Agencement de dosage de liquide selon l'une quelconque des revendications 1 à 6, comprenant en outre un filtre passe-bas (7), qui est agencé pour filtrer le signal provenant du deuxième capteur de pression (5).

8. Agencement de dosage de liquide selon l'une quelconque des revendications 1 à 7, dans lequel le système de régulation de pression (15) comprend un premier dispositif comparateur (8), auquel les signaux provenant des capteurs de pression (4, 5) sont délivrés, et qui calcule la chute de pression $\Delta p$, et un deuxième dispositif comparateur (9), qui compare la chute de pression $\Delta p$, générée par le premier dispositif comparateur (8), avec une valeur de référence prédéterminée $\Delta p_{ref}$ pour la chute de pression, dans lequel la sortie dudit deuxième dispositif comparateur (9) représente le signal de régulation pour le régulateur de pression (6).

9. Agencement de dosage de liquide selon l'une quelconque des revendications 1 à 8, comprenant en outre une alarme (11) qui est agencée pour être activée lorsque la deuxième pression $p_2$ mesurée par le deuxième capteur de pression (5) dépasse une certaine valeur prédéterminée $p_{max}$.

10. Agencement de dosage de liquide selon l'une quelconque des revendications 1 à 9, comprenant en outre une alarme (11) qui est agencée pour être activée lorsque le débit mesuré par un capteur de débit (16) dans le système d'alimentation en liquide (3) dépasse certaines valeurs prédéterminées.

11. Appareil d'injection intraveineuse comprenant un agencement de dosage de liquide selon l'une quelconque des revendications 1 à 10.

**FIG 1**

21

22

$d_1$

**FIG 2a**

21

b

**FIG 2b**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4976687 A